# EUROPEAN PATENT SPECIFICATION

(11) **EP 4 452 436 B1**
(45) Date of publication and mention of the grant of the patent: **23.07.2025**
(21) Application number: 22843721.6
(22) Date of filing: 21.12.2022
(51) Int. Cl.: B01D 3/14, B01D 3/36, C07C 253/34, C07C 255/03

(54) **PROCESS FOR RECOVERING AN ACETONITRILE**
VERFAHREN ZUR GEWINNUNG VON ACETONITRIL
PROCÉDÉ DE RÉCUPÉRATION D'UN ACÉTONITRILE

(30) Priority: 22.12.2021 BE 202106037
(43) Date of publication of application: 30.10.2024
(73) Proprietor: DE NEEF CHEMICAL PROCESSING N.V., 2220 Heist-op-den-Berg (BE)
(72) Inventor: VERMEULEN, Geert, 2220 HEIST-OP-DEN-BERG (BE)
(74) Representative: EP&C
(86) International application number: PCT/EP2022/087363
(87) International publication number: WO 2023/118376

(56) References cited:
- WO-A2-2005/044783
- US-A1- 2019 031 581

## Description

### Field of Technology

Acetonitrile is an organic compound, which is often used as a solvent.

### Background

Acetonitrile is used as a solvent in various processes. Many low-value acetonitrile waste streams are disposed of by incineration. This is undesirable. Processes for recovering acetonitrile from low purity waste streams are known in the art. For example, WO2005044783 discloses an improved process for recovering acetonitrile from a dilute aqueous low-grade acetonitrile stream. Figure 2 thereof discusses a process in which use is made of so-called azeotropic distillation, also referred to as pressure-swing distillation, by first distilling at reduced pressure and then distilling at atmospheric pressure.

As mentioned, acetonitrile is used as a solvent in various processes, including the synthesis of polypeptides. The latter results, for example, in a waste stream consisting of acetonitrile, water and peptide residue. The peptide moieties can cause foaming upon recovery of the acetonitrile. Therefore, even using the improved process of WO2005044783, using azeotropic distillation, too much acetonitrile may be entrained in the bottom product of the first distillation tower. That means loss of valuable acetonitrile. Moreover, the waste water is then no longer biologically processable and must be sent to incineration.

US2019031581 discloses a method for separating a mixture of materials A and B by extractive distillation, using an extraction medium having a higher affinity to B than to A, wherein a feed stream comprising A and B is conducted towards the extraction medium in a column, wherein an overhead fraction comprising A and also a liquid fraction comprising B and extraction medium are obtained, the liquid fraction is collected on a collecting tray and heated and partially evaporated in a first indirect heat exchanger, the resultant vapor is released into the column and a non-evaporated proportion of the liquid fraction is collected as sump fraction in the sump of the column, the sump fraction is successively heated in a second indirect heat exchanger and a third indirect heat exchanger and in part evaporated, wherein the resultant vapor is at least in part released into the column, the sump fraction is separated in a stripper into a fraction comprising B and an extraction medium fraction, the extraction medium fraction is used as heating medium for the second heat exchanger, wherein a partially cooled extraction medium fraction is obtained, and an external heating medium is used for the third heat exchanger, and the partially cooled extraction medium fraction is used as heating medium for the first heat exchanger. The materials A and B may be acetonitrile and water. In this process ethylene glycol may be used as extraction medium. This process employs a second column, but this only serves to regenerate the extraction medium. This process therefore differs significantly from the process of WO2005044783.

It is therefore important to further improve the process of WO2005044783, without radical changes in the process and the conditions. In addition, it is essential that an adjustment does not affect the quality of the acetonitrile. For example, the recovered acetonitrile must still be suitable for the synthesis of polypeptides. The problem to be solved by the present invention may therefore be regarded as how to enhance the separation in the distillation column.

This problem has surprisingly been solved.

### Summary of the Invention

The present invention relates to a process according to claim 1.

### Figures

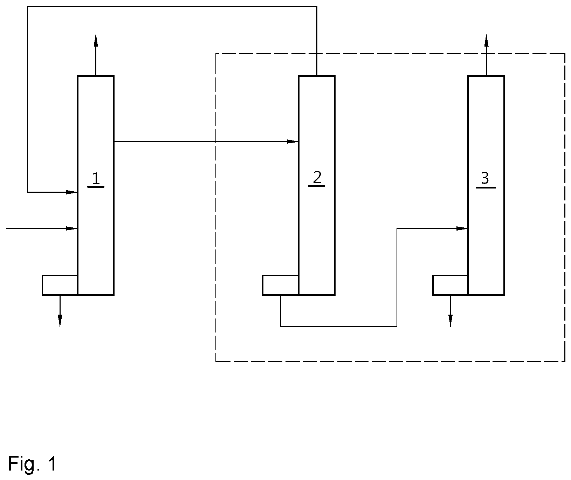

Figure 1 is a schematic representation of a preferred embodiment of the present process.

### Detailed Description of the Invention

The process corresponds to the process as described in WO2005044783.

Preferably, use is made of a liquid alcohol having a boiling point in the range of 180-200°C. This can also be a mixture of alcohols. Various alcohols can be used in the current process.

For example, 2-nonanol or 1-octanol can be used. Preferably, 2-ethylhexanol (2-EH) is used. It is important that the liquid alcohol or mixture thereof is below detection levels in the purified acetonitrile. A common specification on 2-EH for purified acetonitrile is 5 ppm. In the process according to the present invention, the content of 2-EH remains below the detection level determined by means of GC of 1 ppm. The amount of liquid alcohol or mixture thereof is preferably in the range of 0.01-0.5%, more preferably 0.02-0.04%, based on the weight of the acetonitrile stream.

As described in WO2005044783, the pressure during the distillation in the first distillation column may be between 150 and 400 mbar, and more preferably between 200 and 220 mbar. In view of the current modification of the process, the pressure during the distillation in the second distillation column may even be increased from atmospheric to overpressure, for example to 4 bar a, whereby a higher pressure can lead to a reduction of the recycle flow.

In order not to lose valuable acetonitrile, the second acetonitrile/water azeotrope from the second distillation column is preferably recycled to the first distillation column.

For applications in which strict purity requirements are imposed on the acetonitrile, the acetonitrile withdrawn from the second distillation column may be additionally purified or concentrated, preferably by means of an additional distillation and/or with the aid of activated carbon.

The preferred embodiment of the present invention is schematically shown in Figure 1, wherein an acetonitrile stream is added to a distillation column 1. For example, the acetonitrile stream may contain 50% by weight of water. Distillation column 1 carries out a distillation at reduced pressure, preferably between 200-220 mbar. The low-boiling components are withdrawn from the top, and water and high-boiling components are withdrawn from the bottom. As mentioned, it is important that the waste water does not contain too much acetonitrile. The first azeotrope, withdrawn as a side stream, consisting mainly of acetonitrile with about 10% by weight of water, is added to distillation column 2. The second distillation column carries out a distillation at atmospheric pressure or higher, for example at 4 bar a. This results in a second azeotrope withdrawn from the top with about 24% by weight water, and an acetonitrile stream withdrawn from the bottom with less than 0.1% by weight water. The latter is preferably subjected to another distillation in order to separate pure acetonitrile at the top from high-boiling hydrophobic impurities and -if necessary- any salts at the bottom. This is shown schematically in distillation column 3. This second azeotrope is preferably recycled to the distillation column 2.

The invention can be illustrated by the following experiment performed with a solvent stream left over from a polypeptide production. In all cases, use was made of a stream containing approximately 20% by weight of acetonitrile, 1% by weight of high-boiling components and 1% by weight of low-boiling components. The stream contains peptide residues in varying amounts. The remainder of the solvent stream is water.

### Experiment 1

No problems with foaming were experienced in this stream. The waste stream from distillation column 1 therefore contains less than 0.1% by weight of acetonitrile. The amount of high-boiling ingredients has been increased to 1.25% by weight. The amount of low boiling components is below the detection level of 0.01%.

### Experiment 2

In a second stream, problems with foam formation were experienced by peptide residues. The waste stream from distillation column 1 therefore contains approximately 2% by weight of acetonitrile. This waste stream cannot therefore be processed biologically. In addition, about 10% of acetonitrile is lost as a result.

### Experiment 3

To the second stream with foaming peptide residues was added 2-EH. This surprisingly suppressed foaming. The waste stream from distillation column 1 therefore again contains less than 0.1% by weight of acetonitrile.

These experiments show that adding a liquid alcohol with a boiling point in the range 170-220°C suppresses foaming by peptide residues and therefore leads to an improved process.

## Claims

1. A process for recovering acetonitrile from an acetonitrile stream consisting of acetonitrile, 16 to 90 weight percent water, low boiling impurities having a boiling point below the boiling point of acetonitrile/water azeotrope, and high boiling impurities having a boiling point above the boiling point of acetonitrile, wherein the process successively comprises the steps of:
A) introducing the acetonitrile stream into a first distillation column and, by performing a subatmospheric pressure distillation, separating a first acetonitrile/water azeotrope and the low-boiling impurities from the high-boiling impurities, wherein
1) the high-boiling impurities are substantially withdrawn through the bottom of the first distillation column;
2) the low boiling impurities are essentially withdrawn, as vapor, through the top of the first distillation column and
3) the first acetonitrile/water azeotrope and any remaining low-boiling impurities are withdrawn via a side outlet of the first distillation column;
B) introducing the first acetonitrile/water azeotrope withdrawn through a side outlet of the first distillation column into a second distillation column, and by conducting a distillation at atmospheric pressure or higher pressure, separating a second acetonitrile/water -azeotrope of purified acetonitrile wherein the second azeotrope is withdrawn, as vapor, from the top of the second distillation column and acetonitrile is withdrawn from the bottom of the second distillation column,
**characterized in that** a liquid alcohol having a boiling point in the range of 170-220°C has been added to the acetonitrile stream.

2. A process according to claim 1, wherein the liquid alcohol has a boiling point in the range of 180-200°C.

3. A process according to claim 1 or 2, wherein the liquid alcohol is a mixture of alcohols.

4. A process according to any one of claims 1-3, wherein the liquid alcohol is 2-ethylhexanol or a mixture thereof.

5. A process according to any one of claims 1-4, wherein the amount of liquid alcohol or mixture thereof is in the range 0.01-0.5%, preferably 0.02-0.04%, calculated on the weight of the acetonitrile stream.

6. Process according to any one of claims 1-5, wherein the pressure during the distillation in the first distillation column is between 150 and 400 mbar, and more preferably between 200 and 220 mbar.

7. A process according to any one of claims 1-6, wherein the second acetonitrile/water azeotrope from the second distillation column is recycled to the first distillation column.

8. Process according to any one of claims 1-7, wherein the acetonitrile withdrawn from the second distillation column is further purified or concentrated, preferably by means of an additional distillation.

## Patentansprüche

1. Verfahren zur Rückgewinnung von Acetonitril aus einem Acetonitrilstrom, der aus Acetonitril, 16 bis 90 Gew.-% Wasser, niedrigsiedenden Verunreinigungen mit einem Siedepunkt unter dem Siedepunkt von Acetonitril/Wasser-Azeotrop und hochsiedenden Verunreinigungen mit einem Siedepunkt über dem Siedepunkt von Acetonitril besteht, wobei das Verfahren nacheinander die folgenden Schritte umfasst:
A) Eintragen des Acetonitrilstroms in eine erste Destillationskolonne und Abtrennen eines ersten Acetonitril/Wasser-Azeotrops und der niedrigsiedenden Verunreinigungen von den hochsiedenden Verunreinigungen durch Durchführung einer Unterdruck-Destillation, wobei
1) die hochsiedenden Verunreinigungen weitgehend über den Sumpf der ersten Destillationskolonne abgezogen werden;
2) die niedrigsiedenden Verunreinigungen im Wesentlichen als Dampf über den Kopf der ersten Destillationskolonne abgezogen werden und
3) das erste Acetonitril/Wasser-Azeotrop und jegliche verbleibenden niedrigsiedenden Verunreinigungen über einen Seitenabzug der ersten Destillationskolonne abgezogen werden;
B) Eintragen des über einen Seitenabzug der ersten Destillationskolonne abgezogenen ersten Acetonitril/Wasser-Azeotrops in eine zweite Destillationskolonne und Abtrennen eines zweiten Acetonitril/Wasser-Azeotrops von gereinigtem Acetonitril durch Durchführen einer Destillation bei Normaldruck oder höherem Druck, wobei das zweite Azeotrop als Dampf aus dem Kopf der zweiten Destillationskolonne abgezogen wird und Acetonitril aus dem Sumpf der zweiten Destillationskolonne abgezogen wird,
**dadurch gekennzeichnet, dass** ein flüssiger Alkohol mit einem Siedepunkt im Bereich von 170-220 °C zu dem Acetonitrilstrom gegeben wurde.

2. Verfahren nach Anspruch 1, wobei der flüssige Alkohol einen Siedepunkt im Bereich von 180-200 °C aufweist.

3. Verfahren nach Anspruch 1 oder 2, wobei es sich bei dem flüssigen Alkohol um eine Mischung von Alkoholen handelt.

4. Verfahren nach einem der Ansprüche 1-3, wobei es sich bei dem flüssigen Alkohol um 2-Ethylhexanol oder eine Mischung davon handelt.

5. Verfahren nach einem der Ansprüche 1-4, wobei die Menge an flüssigem Alkohol oder einer Mischung davon im Bereich von 0,01-0,5 %, vorzugsweise 0,02-0,04 %, berechnet auf das Gewicht des Acetonitrilstroms, liegt.

6. Verfahren nach einem der Ansprüche 1-5, wobei der Druck während der Destillation in der ersten Destillationskolonne zwischen 150 und 400 mbar und weiter bevorzugt zwischen 200 und 220 mbar liegt.

7. Verfahren nach einem der Ansprüche 1-6, wobei das zweite Acetonitril/Wasser-Azeotrop aus der zweiten Destillationskolonne in die erste Destillationskolonne zurückgeführt wird.

8. Verfahren nach einem der Ansprüche 1 bis 7, wobei das aus der zweiten Destillationskolonne abgezogene Acetonitril weiter gereinigt oder aufkonzentriert wird, vorzugsweise mittels einer zusätzlichen Destillation.

## Revendications

1. Procédé de récupération d'acétonitrile à partir d'un flux d'acétonitrile composé d'acétonitrile, de 16 à 90 % en poids d'eau, d'impuretés à bas point d'ébullition ayant un point d'ébullition au-dessous du point d'ébullition de l'azéotrope acétonitrile/eau, et d'impuretés à point d'ébullition élevé ayant un point d'ébullition au-dessus du point d'ébullition de l'acétonitrile, le procédé comprenant successivement les étapes consistant à :
A) introduire le flux d'acétonitrile dans une première colonne de distillation et, en effectuant une distillation à pression subatmosphérique, séparer un premier azéotrope acétonitrile/eau et les impuretés à bas point d'ébullition des impuretés à point d'ébullition élevé, dans lequel
1) les impuretés à point d'ébullition élevé sont sensiblement éliminées au fond de la première colonne de distillation ;
2) les impuretés à bas point d'ébullition sont pratiquement éliminées, sous forme de vapeur, au sommet de la première colonne de distillation et
3) le premier azéotrope acétonitrile/eau et les impuretés à bas point d'ébullition restantes sont évacués via une sortie latérale de la première colonne de distillation ;
B) introduire le premier azéotrope acétonitrile/eau évacué par une sortie latérale de la première colonne de distillation dans une deuxième colonne de distillation et, en conduisant une distillation à pression atmosphérique ou une pression supérieure, séparer un deuxième azéotrope acétonitrile/eau d'acétonitrile purifié, le deuxième azéotrope étant évacué, sous forme de vapeur, au sommet de la deuxième colonne de distillation et l'acétonitrile étant évacué au fond de la deuxième colonne de distillation,
**caractérisé en ce qu'**un alcool liquide ayant un point d'ébullition compris entre 170 et 220 °C a été ajouté au flux d'acétonitrile.

2. Procédé selon la revendication 1, dans lequel l'alcool liquide a un point d'ébullition compris entre 180 et 200 °C.

3. Procédé selon la revendication 1 ou 2, dans lequel l'alcool liquide est un mélange d'alcools.

4. Procédé selon l'une quelconque des revendications 1 à 3, dans lequel l'alcool liquide est le 2-éthylhexanol ou un mélange contenant celui-ci.

5. Procédé selon l'une quelconque des revendications 1 à 4, dans lequel la quantité d'alcool liquide ou de mélange correspondant est comprise entre 0,01 et 0,5 %, de préférence entre 0,02 et 0,04 %, calculée par rapport au poids du flux d'acétonitrile.

6. Procédé selon l'une quelconque des revendications 1 à 5, dans lequel la pression pendant la distillation dans la première colonne de distillation est comprise entre 150 et 400 mbar, et plus préférablement entre 200 et 220 mbar.

7. Procédé selon l'une quelconque des revendications 1 à 6, dans lequel le deuxième azéotrope acétonitrile/eau provenant de la deuxième colonne de distillation est recyclé vers la première colonne de distillation.

8. Procédé selon l'une quelconque des revendications 1 à 7, dans lequel l'acétonitrile évacué de la deuxième colonne de distillation est purifié ou concentré davantage, de préférence au moyen d'une distillation supplémentaire.
